# EUROPEAN PATENT SPECIFICATION

(11) **EP 0 762 889 B1**
(45) Date of publication and mention of the grant of the patent: **07.05.2003**
(21) Application number: 95916583.8
(22) Date of filing: 17.03.1995
(51) Int. Cl.: A61K 38/18, C12N 15/12, C12N 15/62, A61P 31/00

(54) **HEPARIN-BINDING PROTEIN FOR THE TREATMENT OF SEPSIS**
HEPARIN-BINDING PROTEIN ZUR BEHANDLUNG VON SEPSIS
PROTEINE DE LIAISON DE L'HEPARINE DESTINEE AU TRAITEMENT DE LA SEPTICEMIE

(30) Priority: 21.04.1994 DK 46494; 21.12.1994 DK 145294
(43) Date of publication of application: 19.03.1997
(73) Proprietor: Leukotech A/S, 2100 Copenhagen (DK)
(72) Inventor: FLODGAARD, Hans Jakob Hem, 2900 Hellerup (DK); RASMUSSEN, Poul Baad, 2100 Köbenhavn (DK)
(74) Representative: Hoeiberg, Susanne
(86) International application number: DK9500121
(87) International publication number: WO95028949

(56) References cited:
- WO-A-89/08666
- WO-A-92/02539
- WO-A-93/19087
- US-A- 5 089 274
- DIALOG INFORMATION SERVICES, File 155, Medline, Dialog Accession No. 07793140, Medline Accession No. 91312140, ROGELJ S. et al., "Construction and Expression of Transforming Gene Resulting from Fusion of Basic Fibroblast Growth Factor Gene with Signal Peptide Sequence"; & METHODS ENZYMOL. (UNITED STATES), 1991, 198, p117-24.
- DIALOG INFORMATION SERVICES, File 351, WPIL, Dialog Accession No. 010216610, WPI Accession No. 95-117864/16, MITSUBISHI KASEI CORP., "Heparin Binding Growth Factor Prodn. - by Culturing Transformant Integrated with Heparin Binding Growth Factor in Presence of Sulphated Polysaccharide or Its Agonist"; & JP,A,07 039 388, 10-02-95, 9516 (Basic).
- DIALOG INFORMATION SERVICES, File 155, Medline, Dialog Accession No. 06950917, Medline Accession No. 89252917, KNOERZER W. et al., "Expression of Synthetic Genes Encoding Bovine and Human Basic Fibroblast Growth Factors (bFGFs) in Escherichia Coli"; & GENE (NETHERLANDS), 30 Jan. 1989, 75 (1), p21-30.

### SEQUENCE LISTING

(1) GENERAL INFORMATION:
   (i) APPLICANT:
      (A) NAME: Novo Nordisk A/S
      (B) STREET: Novo Alle
      (C) CITY: Bagsvaerd
      (E) COUNTRY: Denmark
      (F) POSTAL CODE (ZIP): 2880
      (G) TELEPHONE: +45 4444 8888
      (H) TELEFAX: +45 4449 3256
      (I) TELEX: 37304
   (ii) TITLE OF INVENTION: Heparin-Binding Protein for the Treatment of Sepsis
   (iii) NUMBER OF SEQUENCES: 2
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25 (EPO)
(2) INFORMATION FOR SEQ ID NO: 1:
   (i) SEQUENCE CHARACTERISTICS:
      (A) LENGTH: 221 amino acids
      (B) TYPE: amino acid
      (C) STRANDEDNESS: single
      (D) TOPOLOGY: linear
   (ii) MOLECULE TYPE: protein
   (vi) ORIGINAL SOURCE:
      (A) ORGANISM: human
   (xi) SEQUENCE DESCRIPTION: SEQ ID NO: 1:
   Ile Val Gly Gly Arg Lys Ala Arg Pro Arg Gln Phe Pro Phe Leu Ala
   1 5 10 15
   Ser Ile Gln Asn Gln Gly Arg His Phe Cys Gly Gly Ala Leu Ile His
   20 25 30
   Ala Arg Phe Val Met Thr Ala Ala Ser Cys Phe Gln Ser Gln Asn Pro
   35 40 45
   Gly Val Ser Thr Val Val Leu Gly Ala Tyr Asp Leu Arg Arg Arg Glu
   50 55 60
   Arg Gln Ser Arg Gln Thr Phe Ser Ile Ser Ser Met Ser Glu Asn Gly
   65 70 75 80
   Tyr Asp Pro Gln Gln Asn Leu Asn Asp Leu Met Leu Leu Gln Leu Asp
   85 90 95
   Arg Glu Ala Asx Leu Thr Ser Asx Val Thr Ile Leu Pro Leu Pro Leu
   100 105 110
   Gln Asx Ala Thr Val Glu Ala Gly Thr Arg Cys Gln Val Ala Gly Trp
   115 120 125
   Gly Ser Gln Arg Ser Gly Gly Arg Leu Ser Arg Phe Pro Arg Phe Val
   130 135 140
   Asx Val Thr Val Thr Pro Glu Asp Gln Cys Arg Pro Asn Asn Val Cys
   145 150 155 160
   Thr Gly Val Leu Thr Arg Arg Gly Gly Ile Cys Asn Gly Asp Gly Gly
   165 170 175
   Thr Pro Leu Val Cys Glu Gly Leu Ala His Gly Val Ala Ser Phe Ser
   180 185 190
   Leu Gly Pro Cys Gly Arg Gly Pro Asp Phe Phe Thr Arg Val Ala Leu
   195 200 205
   Phe Arg Asp Trp Ile Asp Gly Val Leu Asn Asn Pro Gly
   210 215 220
(2) INFORMATION FOR SEQ ID NO: 2:
   (i) SEQUENCE CHARACTERISTICS:
      (A) LENGTH: 219 amino acids
      (B) TYPE: amino acid
      (C) STRANDEDNESS: single
      (D) TOPOLOGY: linear
   (ii) MOLECULE TYPE: protein
   (vi) ORIGINAL SOURCE:
      (A) ORGANISM: porcine
   (xi) SEQUENCE DESCRIPTION: SEQ ID NO: 2:
   Ile Val Gly Gly Arg Arg Ala Gln Pro Gln Glu Phe Pro Phe Leu Ala
   1               5                   10                  15
   Ser Ile Gln Lys Gln Gly Arg Pro Phe Cys Ala Gly Ala Leu Val His
               20                  25                  30
   Pro Arg Phe Val Leu Thr Ala Ala Ser Cys Phe Arg Gly Lys Asn Ser
           35                  40                  45
   Gly Ser Ala Ser Val Val Leu Gly Ala Tyr Asp Leu Arg Gln Gln Glu
       50                  55                  60
   Gln Ser Arg Gln Thr Phe Ser Ile Arg Ser Ile Ser Gln Asn Gly Tyr
   65                  70                  75                  80
   Asp Pro Arg Gln Asn Leu Asn Asp Val Leu Leu Leu Gln Leu Asp Arg
                   85                  90                  95
   Glu Ala Arg Leu Thr Pro Ser Val Ala Leu Val Pro Leu Pro Pro Gln
               100                 105                 110
   Asx Ala Thr Val Glu Ala Gly Thr Asn Cys Gln Val Ala Gly Trp Gly
           115                 120                 125
   Thr Gln Arg Leu Arg Arg Leu Phe Ser Arg Phe Pro Arg Val Leu Asx
       130                 135                 140
   Val Thr Val Thr Ser Asn Pro Cys Leu Pro Arg Asp Met Cys Ile Gly
   145                 150                 155                 160
   Val Phe Ser Arg Arg Gly Arg Ile Ser Gln Gly Asp Arg Gly Thr Pro
                   165                 170                 175
   Leu Val Cys Asn Gly Leu Ala Gln Gly Val Ala Ser Phe Leu Arg Arg
               180                 185                 190
   Arg Phe Arg Arg Ser Ser Gly Phe Phe Thr Arg Val Ala Leu Phe Arg
           195                 200                 205
   Asn Trp Ile Asp Ser Val Leu Asn Asn Pro Pro
       210                 215

## Description

### FIELD OF INVENTION

The present invention relates to the use of heparin-binding protein in the manufacture of a medicament for the treatment or prevention of diseases or conditions caused by bacterial endotoxin.

### BACKGROUND OF THE INVENTION

Septic shock resulting from a systemic response to serious infection has been increasing in incidence over the last 50 years and is currently the commonest cause of death in intensive care units in the US. The reasons for this increase and high incidence of septic shock are believed to be the increased use of invasive devices such as intravascular catheters, increased use of cytotoxic and immunosuppresive drugs, increased longevity of patients liable to develop sepsis and an increase in infections caused by antibiotic-resistant organisms.

Disorders associated with sepsis are bacteremia (also known as septicemia) characterized by positive blood cultures; sepsis characterized by a systemic response to the infection in the form of tachypnea, tachycardia, hyperthermia or hypothermia; sepsis syndrome in which there is clinical evidence of sepsis and signs of altered organ perfusion in the form of an abnormally increased lactate level, oliguria or acutely altered mental status; early septic shock in which there is clinical evidence of sepsis syndrome as well as hypotension lasting for less than one hour and responsive to conventional therapy; and refractory septic shock in which there is clinical evidence of sepsis syndrome and hypotension lasting for more than one hour despite conventional therapy.

Known mediators of sepsis interact in a complex manner to form a sepsis, or cytokine, cascade initiated at a site of infection or injury (e.g. the abdominal cavity). The initiator of the cytokine cascade (in gram-negative bacteremia which is a common cause of sepsis) is endotoxin (otherwise known as lipopolysaccharide, abbreviated to LPS) released by the bacteria at an infectious or inflammatory site where it induces the release of tumour necrosis factor α (TNF_{α}), interleukin-1, interleukin-6, interleukin-8 and platelet-activating factor (PAF) from macrophages and other cells. After release of TNF_{α}, interleukin-1 and PAF, arachidonic acid is metabolized to form leukotrienes, thromboxane A₂ and prostaglandins. Interleukin-1 and interleukin-6 activate T-cells to produce interferon-γ, interleukin-2, interleukin-4 and granulocyte-monocyte colony-stimulating factor. Neutrophil leukocytes may be activated directly by most of these mediators. Neutrophil-induced damage may thus occur during degranulation by the release of oxygen free radicals and lysosomal enzymes, during aggregation at the infectious or inflammatory site.

While potentially capable of triggering the cytokine cascade, the presence of endotoxin or other sepsis mediators at infectious or inflammatory foci need not in itself lead to sepsis, as the cytokine cascade has certain inherent safeguards against uncontrolled activation of the cytokine cascade. Thus, prostaglandin may inhibit the release of cytokines from macrophages, and macrophages may be able to suppress T cells. Clinical symptoms of sepsis would appear to occur when too much endotoxin or other mediators have been released or when the mediators capable of interfering with the cytokine cascade are not present; e.g. when the immune system is attenuated. For a more detailed description of the progression of septic disorders and the mediators involved therein, *vide* R.C. Bone, "The Pathogenesis of Sepsis", Ann. Int. Med. 115, 1991, pp. 457-469.

The continued high mortality and morbidity attributable to gram-negative sepsis has prompted an intensive search for therapeutic agents capable of counteracting the potentially lethal effects of circulating bacterial LPS. Numerous papers report a significant therapeutical effect of high doses of intravenously administered immunoglobulin. The treatment, however, requires IgG derived from the plasma of donors screened for naturally occurring high levels of antibodies to core LPS or from very large pools of donors (>1000). Monoclonal antibodies against LPS which have also been suggested for the treatment of bacteremia (e.g. WO88/03211) have shown little or no effect, probably because they do not inhibit the cytokine cascade induced by LPS.

The covalent structure of two closely related proteins isolated from peripheral neutrophil leukocytes of human and porcine origin have recently been determined (cf. H. Flodgaard et al., Eur. J. Biochem. 197, 1991, pp. 535-547; J. Pohl et al., FEBS Lett. 272, 1990, p. 200 ff.). Both proteins show a high similarity to neutrophil elastase, but owing to selective mutations of the active serine 195 and histidine 57 (chymotrypsin numbering (B.S. Hartley, "Homologies in Serine Proteinases", Phil. Trans. Roy. Soc. Series 257, 1970, p. 77 ff.)) the proteins lack protease activity. The proteins have been named human heparin-binding protein (hHBP) and porcine heparin-binding protein (pHBP), respectively, owing to their high affinity for heparin; Schafer et al. (W.M. Schafer et al., Infect. Immun. 53, 1986, p. 651 ff.) have named the protein cationic antimicrobial protein (CAP37) due to its antimicrobial activity. The protein has also been shown to be chemotactic for monocytes over the range 1.3 x 10⁻⁹ M - 10⁻⁸ M (H.A. Pereira et al., J. Clin.Invest. 85, 1990, p.1468 ff.), consistent with the results apparent from Flodgaard et al., op. cit..

Furthermore, HBP has been shown to mediate detachment and contraction of endothelial cells and fibroblasts when added to such cells grown in monolayer culture. HBP also stimulates monocyte survival and thrombospondin secretion (E. Østergaard and H. Flodgaard, J. Leukocyte Biol. 51, 1992, p 316 ff).

From the azurophil granules, a protein with the first 20 N-terminal amino acid residues identical to those of. hHBP and CAP37 called azurocidin has also been isolated (J.E. Gabay et al., Proc. Natl. Acad. Sci. USA 86, 1989, p. 5610 ff.; C.G. Wilde et al., J. Biol. Chem. 265, 1990, p. 2038 ff.) and its antimicrobial properties have been reported (D. Campanelli et al., J. Clin. Invest. 85, 1990, p. 904 ff.).

The presence of hHBP in the neutrophil leucocytes and the fact that 89% of CAP37 (which is identical to hHBP) is released when the leucocytes are phagocytosing Staph. aureus (H.A. Pereira et al., op. cit.) indicate that a function of hHBP could be its involvement in the inflammatory process since the protein is apparently released from activated neutrophils. Pereira et al., op. cit., suggested a function of CAP37 to be at the site of inflammation where it could specifically attract monocytes and thus be one of the factors responsible for the influx of monocytes in the second wave of inflammation. Østergaard and Flodgaard, op. cit., suggest that, in addition to being important for the recruitment of monocytes, HBP might play a key role in the mechanism of neutrophil as well as monocyte extravasation.

The structure of HBP appears from WO 89/08666 and H. Flodgaard et al., op. cit. HBP has otherwise been termed CAP37 (cf. WO 91/00907) and azurocidin (cf. C.G. Wilde et al., J. Biol. Chem. 265, 1990, p. 2038).

### SUMMARY OF THE INVENTION

It has not previously been suggested that HBP may be used in the treatment of diseases or conditions caused by the presence of bacterial endotoxin at sites of infection or injury.

Accordingly, the present invention relates to the use of heparin-binding protein which, in glycosylated form, has an apparent molecular weight of 28 kD (as determined by SDS-PAGE under reducing conditions) the HBP being producible in the azurophil granules of polymorphonuclear leukocytes, or an analogue or a peptide fragment of HBP according to SEQ 1 or 2, the peptide being capable of binding the lipid A portion of LPS, for the manufacture of a medicament for the prevention or treatment of sepsis, gram-negative sepsis, septic shock, or disseminated intravascular coagulation.

HBP has been found to bind to LPS, cf. H.A. Pereira et al., Proc. Natl. Acad. Sci. USA 90, 1993, pp. 4733-4737, where this ability is linked to the antibacterial effect of the protein. It is reported that the bactericidal effect of HBP is reduced at pH 7 (i.e. physiological pH). Furthermore, septic conditions are not caused by the gram-negative bacteria as such, but by the endotoxin released from dead bacterial cells activating the CD14 receptor on monocytes, thereby inducing the cytokine cascade. It would therefore seem that the antibacterial effect of HBP is of no importance for the treatment of septic conditions. In this context, it should be noted that LPS binding in itself is not indicative of any ability to neutralize the cytokine cascade-inducing effects of endotoxin, as evidenced by the fact that antibodies raised against LPS have little or no effect in this respect, cf. E.Th. Rietschel and M. Schlaak, Immun.lnfect. 21, 1993, pp. 25-36.

While the prior published efforts at combating septic conditions have concentrated on neutralizing endotoxin in the circulation of sepsis patients (e.g. as suggested in WO 93/19087 in which CAP37 (i.e. HBP) is suggested for the treatment of endotoxemia by binding to and providing clearance of LPS from the circulation), most of these efforts have been in vain i.a. because sepsis patients do not generally have elevated levels of circulating endotoxin. HBP may, however, be effective at local inflammatory or infectious sites after septic phenomena have manifested themselves, that is at post-endotoxemic events. It is at present assumed that HBP may exert its beneficial effect not only by binding specifically and with high affinity to Lipid A (the toxic portion of LPS) thereby inhibiting continued induction of the cytokine cascade, but also by protecting monocytes from apoptosis resulting from stress injury caused by the release of oxygen free radicals and cytokines from phagocytes (e.g. neutrophils, monocytes, macrophages) as part of the body's defence mechanisms against injury and infections.

Monocytes migrate to sites of injury or infection where they phagocytose infectious microorganisms and harmful substances such as endotoxin. HBP may also stimulate the phagocytic effect of monocytes. The ability of HBP to mobilise monocytes would appear to be dependent on its ability to bind LPS in a manner which has not yet been clarified.

Furthermore, it has surprisingly been established that HBP binds irreversibly to LPS at 37-45°C (i.e. normal to hyperthermic body temperature). This, too, may have an effect on the neutralization of LPS by HBP.

### DETAILED DESCRIPTION OF THE INVENTION

The HBP to be used for the manufacture of a medicament may suitably be of mammalian, in particular human or porcine, origin. In particular, the HBP is human HBP with the amino acid sequence shown in the Sequence Listing as SEQ ID NO:1, or porcine HBP with the amino acid sequence shown in the Sequence Listing as SEQ ID NO:2, or a functional analogue or peptide fragment thereof capable of binding the lipid A portion of LPS. Examples of such functional analogues include derivatives of the native protein obtained by addition of one or more amino acid residues to either or both the C- or N-terminal end of the native protein, substitution of one or more amino acid residues at either or both ends of the native protein, deletion of one or more amino acid residues at either or both ends of the native protein or at one or more sites within the amino acid sequence, or insertion of one or more amino acid residues at one or more sites in the native amino acid sequence. The term is specifically intended to include peptide fragments of HBP, in particular fragments with a similar ability as native HBP to reduce the LPS-induced cytokine cascade from human mononuclear cells. An example of such a fragment capable of binding the lipid A proteion of LPS is a peptide fragment comprising amino acid residues 20-53, in particular amino acid residues 26-42 of SEQ ID NO:1 or amino acid residues 20-53 of SEQ ID NO:2.

A DNA sequence encoding HBP may be prepared synthetically by established standard methods, e.g. the phosphoamidite method described by S.L. Beaucage and M.H. Caruthers, Tetrahedron Letters 22, 1981, pp. 1859-1869, or the method described by Matthes et al., EMBO Journal 3, 1984, pp. 801-805. According to the phosphoamidite method, oligonucleotides are synthesized, e.g. in an automatic DNA synthesizer, purified, annealed, ligated and cloned in suitable vectors.

The DNA sequence may also be of genomic or cDNA origin, for instance obtained by preparing a genomic or cDNA library and screening for DNA sequences coding for all or part of HBP by hybridization using synthetic oligonucleotide probes in accordance with standard techniques (cf. Sambrook et al., Molecular Cloning: A Laboratory Manual, 2nd Ed., Cold Spring Harbor, 1989). The DNA sequence may also be prepared by polymerase chain reaction using specific primers, for instance as described in US 4,683,202 or R.K. Saiki et al., Science 239, 1988, pp. 487-491.

The DNA sequence is then inserted into a recombinant expression vector which may be any vector which may conveniently be subjected to recombinant DNA procedures. The choice of vector will often depend on the host cell into which it is to be introduced. Thus, the vector may be an autonomously replicating vector, i.e. a vector which exists as an extrachromosomal entity, the replication of which is independent of chromosomal replication, e.g. a plasmid. Alternatively, the vector may be one which, when introduced into a host cell, is integrated into the host cell genome and replicated together with the chromosome(s) into which it has been integrated.

In the vector, the DNA sequence encoding HBP should be operably connected to a suitable promoter sequence. The promoter may be any DNA sequence which shows transcriptional activity in the host cell of choice and may be derived from genes encoding proteins either homologous or heterologous to the host cell. Examples of suitable promoters for directing the transcription of the DNA encoding HBP in mammalian cells are the SV 40 promoter (Subramani et al., Mol. Cell Biol. 1, 1981, pp. 854-864), the MT-1 (metallothionein gene) promoter (Palmiter et al., Science 222, 1983, pp. 809-814) or the adenovirus 2 major late promoter. A suitable promoter for use in insect cells is the polyhedrin promoter (Vasuvedan et al., FEBS Lett. 311, 1992, pp. 7-11). Suitable promoters for use in yeast host cells include promoters from yeast glycolytic genes (Hitzeman et al., J. Biol. Chem. 255, 1980, pp. 12073-12080; Alber and Kawasaki, J. Mol. Appl. Gen. 1, 1982, pp. 419-434) or alcohol dehydrogenase'genes (Young et al., in Genetic Engineering of Microorganisms for Chemicals (Hollaender et al, eds.), Plenum Press, New York, 1982), or the TPI1 (US 4, 599, 311) or ADH2-4c (Russell et al., Nature 304, 1983, pp. 652-654) promoters. Suitable promoters for use in filamentous fungus host cells are, for instance, the ADH3 promoter (McKnight et al., The EMBO J. 4, 1985, pp. 2093-2099) or the tpiA promoter.

The DNA sequence encoding HBP may also be operably connected to a suitable terminator, such as the human growth hormone terminator (Palmiter et al., op. cit.) or (for fungal hosts) the TPI1 (Alber and Kawasaki, op. cit.) or ADH3 (McKnight et al., op. cit.) promoters. The vector may further comprise elements such as polyadenylation signals (e.g. from SV 40 or the adenovirus 5 Elb region), transcriptional enhancer sequences (e.g. the SV 40 enhancer) and translational enhancer sequences (e.g. the ones encoding adenovirus VA RNAs).

The recombinant expression vector may further comprise a DNA sequence enabling the vector to replicate in the host cell in question. An examples of such a sequence (when the host cell is a mammalian cell) is the SV 40 origin of replication. The vector may also comprise a selectable marker, e.g. a gene the product of which complements a defect in the host cell, such as the gene coding for dihydrofolate reductase (DHFR) or one which confers resistance to a drug, e.g. neomycin or hygromycin.

The procedures used to ligate the DNA sequences coding for HBP, the promoter and the terminator, respectively, and to insert them into suitable vectors containing the information necessary for replication, are well known to persons skilled in the art (cf., for instance, Sambrook et al., op.cit.).

The host cell into which the expression vector is introduced may be any cell which is capable of producing HBP and is preferably a eukaryotic cell, such as invertebrate (insect) cells or vertebrate cells, e.g. Xenopus laevis oocytes or mammalian cells, in particular insect and mammalian cells. Examples of suitable mammalian cell lines are the COS (e.g. ATCC CRL 1650), BHK (e.g. ATCC CRL 1632, ATCC CCL 10) or CHO (e.g. ATCC CCL 61) cell lines. Methods of transfecting mammalian cells and expressing DNA sequences introduced in the cells are described in e.g. Kaufman and Sharp, J. Mol. Biol. 159, 1982, pp. 601-621; Southern and Berg, J. Mol. Appl. Genet. 1, 1982, pp. 327-341; Loyter et al., Proc. Natl. Acad. Sci. USA 79, 1982, pp. 422-426; Wigler et al., Cell 14, 1978, p. 725; Corsaro and Pearson, Somatic Cell Genetics 7, 1981, p. 603, Graham and van der Eb, Virology 52, 1973, p. 456; and Neumann et al., EMBO J. 1, 1982, pp. 841-845.

Alternatively, fungal cells (including yeast cells) may be used as host cells. Examples of suitable yeast cells include cells of Saccharomyces spp. or Schizosaccharomyces spp., in particular strains of Saccharomyces cerevisiae. Examples of other fungal cells are cells of filamentous fungi, e.g. Aspergillus spp. or Neurospora spp., in particular strains of Aspergillus oryzae or Aspergillus niger. The use of Aspergillus spp. for the expression of proteins is described in, e.g., EP 238 023.

The medium used to culture the cells may be any conventional medium suitable for growing mammalian cells, such as a serum-containing or serum-free medium containing appropriate supplements, or a suitable medium for growing insect, yeast or fungal cells. Suitable media are available from commercial suppliers or may be prepared according to published recipes (e.g. in catalogues of the American Type Culture Collection).

The HBP produced by the cells may then be recovered from the culture medium by conventional procedures including separating the host cells from the medium by centrifugation or filtration, precipitating the proteinaceous components of the supernatant or filtrate by means of a salt, e.g. ammonium sulphate, purification by a variety of chromatographic procedures, e.g. ion exchange chromatography, affinity chromatography, or the like.

It has surprisingly been found that particularly good yields of HBP are obtained when HBP is expressed as pro-HBP. Accordingly, in a specific embodiment, the present invention relates to a process for producing HBP, wherein host cells containing a DNA sequence encoding mature HBP preceded by an N-terminal extension are cultured in a suitable culture medium under conditions permitting expression of HBP, and the resulting HBP is recovered from the culture medium as N-terminally extended HBP.

The N-terminal extension may be a sequence of from about 5 to about 25 amino acid residues, in particular from about 8 to about 15 amino acid residues. The nature of the amino acid residues in the N-terminal sequence is not believed to be critical. The N-terminal extension may suitably be the propeptide of HBP with the amino acid sequence Gly-Ser-Ser-Pro-Leu-Asp.

In order to facilitate production of mature HBP, it is generally preferred that the DNA sequence encoding N-terminally extended HBP includes a DNA sequence encoding a protease cleavage site located between the DNA sequence encoding the N-terminal extension and the DNA sequence encoding mature HBP. Examples of suitable protease cleavage sites are an enterokinase cleavage site with the amino acid sequence (Asp)₄-Lys or a Factor Xa cleavage site with the amino acid sequence Ile-Glu-Gly-Arg.

After recovery from the culture medium, the N-terminally extended HBP may advantageously be cleaved with a suitable protease to produce mature (and active) HBP. Examples of suitable enzymes are enterokinase or Factor Xa.

The host cells used for producing HBP are favourably insect cells, e.g. cells of *Lepidoptera* or *Drosophila*. In this case, the cells may suitably be transfected with a baculovirus vector, e.g. as described in US 4,745,051 or WO 92/01801.

It has furthermore been found advantageous to produce HBP in the presence of heparin or another sulphated polysaccharide in order to avoid binding of HBP to lipopolysaccharide present in the culture medium. Accordingly, in a specific embodiment, the present invention relates to a process for producing HBP, wherein host cells containing a DNA sequence encoding HBP are cultured in a suitable culture medium containing a sulphated polysaccharide under conditions permitting expression of HBP, and the resulting HBP is recovered from the culture medium. The HBP will bind to the sulphated polysaccharide present in the culture medium and should subsequently be released from the polysaccharide under suitable conditions, e.g. by eluting HBP with a salt such as sodium chloride.

The sulphated polysaccharide is preferably heparin although other sulphated polysaccharides such as heparan sulphate, dextran sulphate, dermatan sulphate, pentosan polysulphate or protamine sulphate may also be employed. The sulphated polysaccharide is preferably immobilized on an inert carrier to facilitate the separation of HBP bound to the polysaccharide from the culture medium. The inert carrier may, for instance, be agarose (e.g. Sepharose).

In this embodiment, too, the DNA sequence encoding mature HBP may be preceded by an N-terminal extension as described above.

In a further embodiment, the present invention relates to a process for producing HBP, wherein host cells containing a DNA sequence encoding mature HBP preceded by and fused to a DNA sequence encoding another protein are cultured in a suitable culture medium under conditions permitting expression of a fusion protein of HBP and the other protein, and the resulting fusion protein is recovered from the culture medium. The other protein may, for instance be Factor VIII light chain (the DNA sequence coding for FVIII light chain may, for instance, be obtained as described in EP 232 112).

In this embodiment, too, it may be an advantage to introduce a DNA sequence encoding a protease cleavage site, as described above, located between the N-terminal extension and the mature HBP.

The use of HBP as a medicament may typically be in a form suited for local or systemic injection or infusion and may, as such, be formulated with sterile water or an isotonic saline or glucose solution. The medicament may be sterilized by conventional sterilization techniques which are well known in the art. The resulting aqueous solutions may be packaged for use or filtered under aseptic conditions and lyophilized, the lyophilized preparation being combined with the sterile aqueous solution prior to administration. The medicament may contain pharmaceutically acceptable auxiliary substances as required to approximate physiological conditions, such as buffering agents, tonicity adjusting agents and the like, for instance sodium acetate, sodium lactate, sodium chloride, potassium chloride, calcium chloride, etc. The concentration of HBP used by the invention may vary widely, i.e. from less than about 0.5%, such as from 1%, to as much as 15-20% by weight. A unit dosage of the HBP of the medicament may typically contain from about 10 mg to about 1 g of HBP.

Use of the present HBP₅ is contemplated to be advantageous to use for therapeutic applications such as treatment of sepsis, septic shock, disseminated intravascular coagulation or Meningococcal meningitis. The present use may be particularly for local application at sites of inflammation or infection (e.g. in the abdominal cavity), since it is vital to protect monocytes migrating to such sites and ensure that they are able to internalize endotoxin and infectious bacteria. A daily dosage of HBP of 0.1-100 mg/kg body weight is at present contemplated to be suitable, dependent on the severity of the condition to be treated and the patient's condition.

In the use of the present invention the medicament may additionally comprise an antimicrobial agent to treat the causative bacterial infection. Examples of suitable antimicrobial agents are beta-lactam antibiotics (e.g. penicillin), aminoglycosides, tetracyclines, bacitracin, polymyxin, sulfonamides, nitrofurans, nalidixic acid, etc. For systemic application, the medicament may also contain heparin or another sulfated glucosaminoglycan as an anticoagulant agent since it has surprisingly been found that heparin does not inhibit the ability of HBP to bind to LPS. Apart from acting as an anticoagulant, the presence of heparin may also lead to a longer plasma half-life of HBP as it prevents HBP bind to glucosaminoglycans on the vascular endothelium.

### BRIEF DESCRIPTION OF THE DRAWINGS

The present invention is further illustrated in the following examples with reference to the appended drawings, wherein
Fig. 1 shows the DNA sequence and derived amino acid sequence of a 901 bp EcoRI-XbaI fragment of plasmid pKFN-1783, encoding hHBP;
Fig. 2 shows the DNA sequence and derived amino acid sequence of a 772 bp BamHI-HindIII fragment inserted into plasmid pSX221, encoding hHBP;
Fig. 3 is a graph showing cytokine levels in blood mononuclear cells treated with 10 µg/ml HSA or varying levels of HBP in the presence of 10 ng/ml LPS; and
Fig. 4 is a graph showing cytokine levels in blood mononuclear cells treated with 10 µg/ml HSA or varying levels of HBP in the presence of 100 ng/ml LPS.

### Example 1

### Production of Human Heparin Binding Protein from yeast strain KFN-1775.

### General methods.

Standard DNA techniques were carried out as described (Sambrook, J., Fritch, E.F., and Maniatis, T. (1989) Molecular Cloning: A Laboratory Manual, 2nd edn., Cold Spring Harbor Laboratory Press, Cold Spring Harbor, NY). Synthetic oligonucleotides were prepared on an automatic DNA synthesizer (380B, Applied Biosystems) using commercially available reagents. DNA sequence determinations were performed by the dideoxy chain-termination technique ( Sanger, F., Micklen, S., and Coulson, A.R., Proc. Natl. Acad. Sci. USA 74 (1977) 5463-5467). Polymerase chain reactions ( PCR) were performed on a DNA Thermal Cycler ( Perkin Elmer Cetus).

N-terminal amino acid sequence analysis was obtained by automated Edman degradation using an Applied Biosystems 477A gas-phase sequencer. Analysis by on-line reverse phase HPLC was performed for the detection and quantitation of the liberated PTH amino acids from each sequencer cycle.
1 µl (10⁶ pfu) of a human bone marrow cDNA library (Clontech, Palo Alto, CA, U.S.A.) was used as a template in a PCR reaction containing 100 pmole each of the primers NOR-3176 (GTTGGAATTCAT (A/T/C)CA(A/G)AA(T/C)CA(A/G)GGN(A/C)G) and NOR-3174 ( CA(T/C)TG(A/G)TC(T/C)TCNGGNGT), where N is a mixture of all four nucleotides. The 5' part of forward primer NOR-3176 contains an EcoRI site and the 3' part corresponds to amino acid residues no. 18-23 and reverse primer NOR-2174 corresponds to amino acid residues no. 149-154 in the amino acid sequence of hHBP [Flodgaard, H., Østergaard, E., Bayne, S., Svendsen, A., Thomsen, J., Engels, M., and Wollmer, A. (1991) Eur. J. Biochem. 197, 535-547].

The PCR reaction was performed in a 100µl volume using a commercial kit ( GeneAmp, Perkin Elmer Cetus) and the following cycle: 94° for 1 min, 50° for 2 min, and 72° for 3 min. After 35 cycles a final cycle was performed in which the 72° step was maintained for 10 min. The PCR product, a 420 bp fragment, was isolated by electrophoresis on a 1 % agarose gel.

Similarly primers NOR-3173 (ACNCCNGA(A/G)GA(T/C)CA(A/G)TG) and NOR-3175 (GGTTTCTAGATTATCCCGG(A/G)TT(A/G)TTNA(A/G)NACNCC) were used with the same cDNA template as described above in a PCR reaction. Forward primer NOR-3173 is complementary to NOR-2174 and reverse primer NOR-3175 corresponds to amino acid residues 215-221 in the amino acid sequence of HBP followed by a stop codon and an XbaI site. The PCR reaction was performed as described above and the product, a 232 bp fragment was isolated by agarose gel electrophoresis.
The two PCR fragments described above were joined by overlay extension PCR [Horton, R. M., Hunt, H. D., Ho, S. N., Pullen, J. K., and Pease, L. R. (1989) Gene 77, 61-68] using NOR-3176 and NOR-3175 as end primers. The resulting 635 bp fragment was isolated and then digested with EcoRI and XbaI giving a 621 bp fragment, which was ligated to the 2.8 kb EcoRI-XbaI fragment from plasmid pTZ19R (Mead, D.A., Szczesna-Skorupa, E. and Kemper, B., Prot. Engin. 1 (1986) 67-74).
The ligation mixture was used to transform a competent E. coli strain (r⁻, m⁺) selecting for ampicillin resistance. DNA sequencing showed that plasmid pKFN-1726 from one of the resulting colonies encoded the expected hHBP₁₈₋₂₂₁ amino acid sequence.
Plasmid pKFN-1726 was digested with PstI and XbaI and the resulting 329 bp fragment was isolated and used as described later.

The N-terminal part of HPB was isolated as a 379 bp fragment by PCR using the same cDNA template as described above and primers MHJ-206 (GCTGAGAGATTGGAGAAGAGAATCGTTGGCGGCCGGAAGGCGAG) and MHJ-200 (CGGCTTCCACCGTGGCGTTCTG). The 3' part of MHJ-206 is identical to the N-terminal encoding part of the humane HBP gene, and the 5' part of MHJ-206 is identical to the C-terminal part of the hybrid yeast leader gene described earlier [Thim, L., Norris, K., Norris, F., Nielsen, P. F., Bjørn, S. E., Christensen, and Petersen, J. (1993) FEBS Lett. 318, 345-352].

In-frame fusion of the hybrid leader gene and the 379 bp PCR fragment encoding the N-terminal part of HBP was obtained by overlay extension PCR [Horton, R. M., Hunt, H. D., Ho, S. N., Pullen, J. K., and Pease, L. R. (1989) Gene 77, 61-68]. The product was digested with EcoRI and PstI and isolated as a 572 bp fragment.

The 572 bp EcoRI-PstI fragment and the 329 bp PstI-XbaI fragment described above were ligated to the 2.8 kb EcoRI-XbaI fragment from plasmid pTZ19R (Mead, D.A., Szczesna-Skorupa, E. and Kemper, B., Prot. Engin. 1 (1986) 67-74). The ligation mixture was used to transform a competent E. coli strain (r⁻, m⁺) selecting for ampicillin resistance. Plasmid pKFN-1780 from one of the resulting colonies was selcted for further use.

Plasmid pKFN-1780 was digested with EcoRI and XbaI. The resulting 901 bp fragment was ligated to the 9.5 kb NcoI-XbaI fragment from pMT636 and the 1.4 kb NcoI-EcoRI fragment from pMT636. Plasmid pMT636 is described in International Patent application No. WO 89/01968.

pMT636 is an E. coli - S. cerevisiae shuttle vector containing the Schizosaccharomyces pombe TPI gene (POT) (Russell, P.R., Gene 40 (1985) 125-130), the S. cerevisiae triosephosphate isomerase promoter and terminator, TPIp and TPI_{T} (Alber, T., and Kawasaki, G. J. Mol. Appl. Gen. 1 (1982), 419-434).

The ligation mixture was used to transform a competent E. coli strain (r⁻, m⁺) selecting for ampicillin resistance. DNA sequencing showed that plasmids from the resulting colonies contained the correct DNA sequence for human HPB correctly fused to the synthetic yeast signal-leader gene.

One plasmid pKFN-1783 was selected for further use.

The expression cassette of plasmid pKFN-1783 contains the following sequence:
TPIp - signal-leader - HBP - TPI_{T}

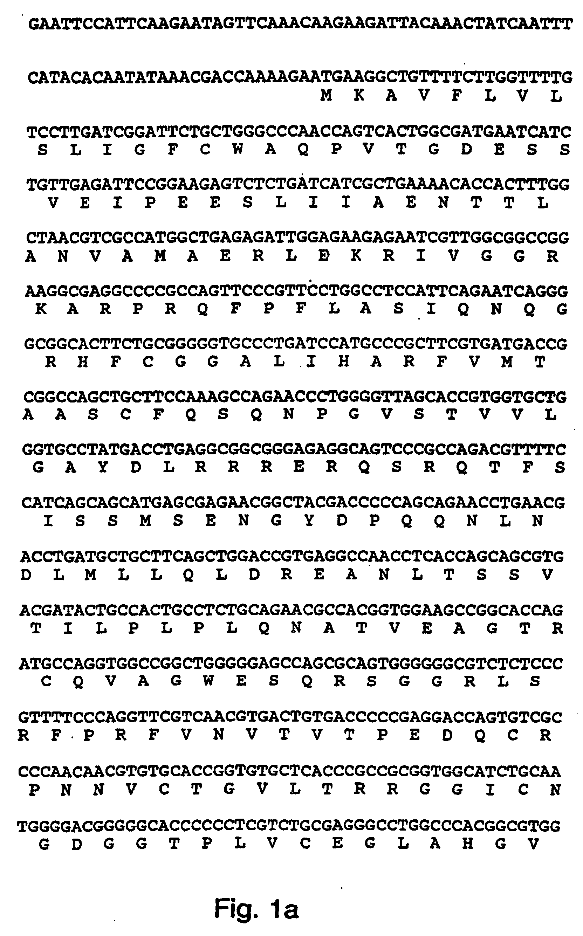

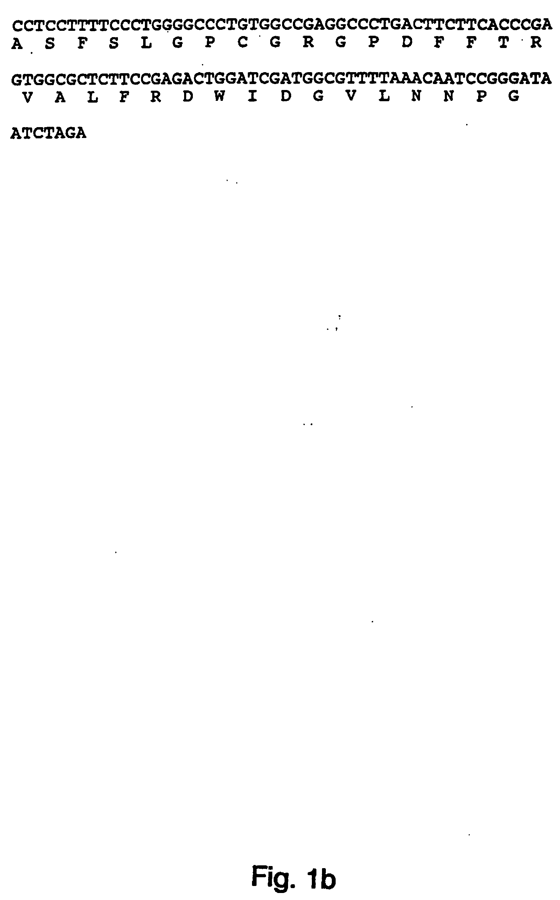

The DNA sequence of the 901 bp EcoRI-XbaI fragment from pKFN-1783 is shown in Fig. 1.

Yeast transformation: S. cerevisiae strain MT663 (E2-7B XE11-36 a/α, Δtpi/Δtpi, pep 4-3/pep 4-3) was grown on YPGaL (1% Bacto yeast extract, 2% Bacto peptone, 2% galactose, 1% lactate) to an O.D. at 600 nm of 0.6.

100 ml of culture was harvested by centrifugation, washed with 10 ml of water, recentrifugated and resuspended in 10 ml of a solution containing 1.2 M sorbitol, 25 mM Na₂EDTA pH = 8.0 and 6.7 mg/ml dithiotreitol. The suspension was incubated at 30°C for 15 minutes, centrifuged and the cells resuspended in 10 ml of a solution containing 1.2 M sorbitol, 10 mM Na₂EDTA, 0.1 M sodium citrate, pH = 5.8, and 2 mg Novozym® 234. The suspension was incubated at 30°C for 30 minutes, the cells collected by centrifugation, washed in 10 ml of 1.2 M sorbitol and 10 ml of CAS (1.2 M sorbitol, 10 mM CaCl₂, 10 mM Tris HCl (Tris = Tris(hydroxymethyl)aminomethane) pH = 7.5) and resuspended in 2 ml of CAS. For transformation, 0.1 ml of CAS-resuspended cells were mixed with approx. 1 µg of plasmid pKFN-1783 and left at room temperature for 15 minutes. 1 ml of (20% polyethylene glycol 4000, 20 mM CaCl₂, 10 mM CaCl₂, 10 mM Tris HCl, pH = 7.5) was added and the mixture left for a further 30 minutes at room temperature. The mixture was centrifuged and the pellet resuspended in 0.1 ml of SOS (1.2 M sorbitol, 33% v/v YPD, 6.7 mM CaCl₂, 14 µg/ml leucine) and incubated at 30°C for 2 hours. The suspension was then centrifuged and the pellet resuspended in 0.5 ml of 1.2 M sorbitol. Then, 6 ml of top agar (the SC medium of Sherman et al., (Methods in Yeast Genetics, Cold Spring Harbor Laboratory (1982)) containing 1.2 M sorbitol plus 2.5% agar) at 52°C was added and the suspension poured on top of plates containing the same agar-solidified, sorbitol containing medium.

Transformant colonies were picked after 3 days at 30°C, reisolated and used to start liquid cultures. One such transformant KFN-1775 was selected for further characterization.

Fermentation: Yeast strain KFN-1775 was grown on YPD medium ( 1% yeast extract, 2% peptone ( from Difco Laboratories), and 3% glucose). A 1 liter culture of the strain was shaken at 30°C to an optical density at 650 nm of 24. After centrifugation the supernatant was isolated.

HBP was purified from the supernatant substantially as described in WO 89/08666. Amino acid sequence analysis of residues 1-20 showed identity of the N-terminal sequence to SEQ ID NO: 1.

### Example 2

In order to express HBP (as proform) in insect cells using the Baculovirus system the following PCR primers were made MHJ 2087 codes for a BamHI site, the initiation codon and the prepro part of the human cDNA (Morgan, J.G. et al. (1991), J.Immun., 147 (3210-3214)) followed by the first 20 nucleotides from the beginning of the mature part of the gene on pKFN1780.

MHJ 2089 is complementary to the last 8 codons from the coding part of the HBP gene on pKFN1780 plus two extra codons according to the cDNA sequence cited above. It ends in a Hind III site.

PCR was performed using the two primers and pKFN1780 as template following the scheme:
3 cycles 95°C 60 sec, 50°C 120 sec, 72°C 120 sec
12 cycles 95°C 30 sec, 65°C 60 sec, 72°C 90 sec

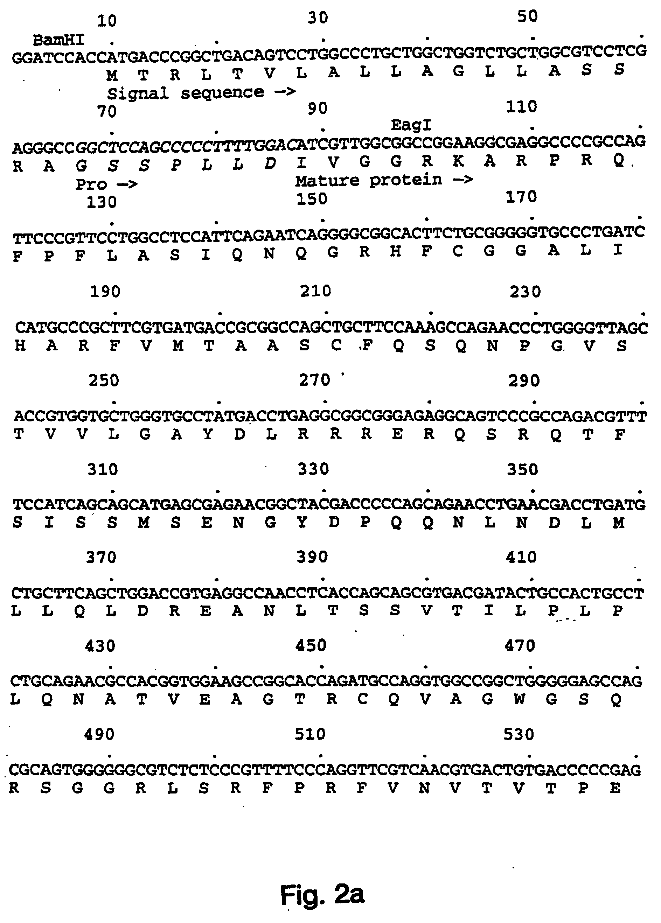

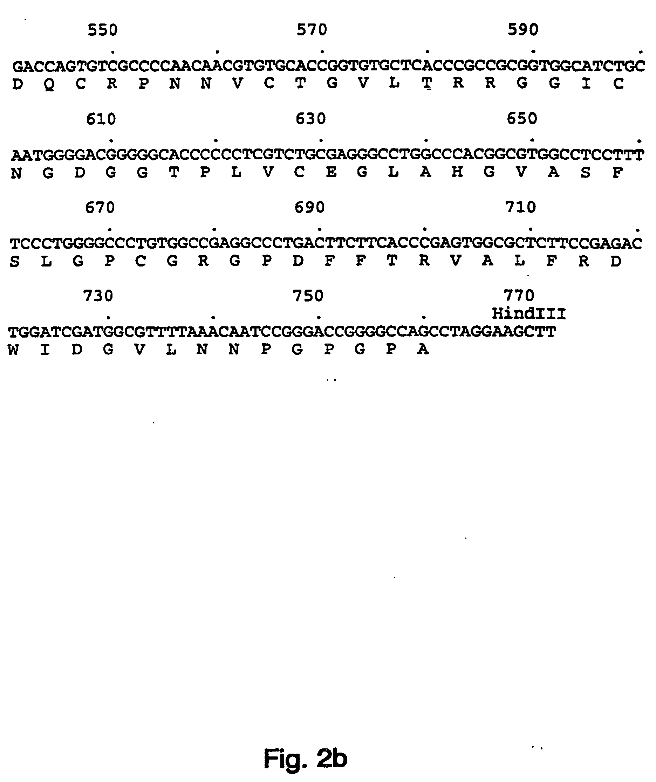

The PCR product, a 760 bp fragment, was isolated by electrophoresis on a 1% agarose gel, cut with BamHI and HindIII, and inserted into pSX221 cut with the same two enxymes. (pSX221 is a derivative of pUC19 (Yannisch-Perron, C. et al. (1985) GENE 33, 103-119). The cloned DNA was verified by sequencing and the BamHI-HindIII fragment (shown in Fig. 2) was cut out, isolated, and inserted into pBlueBacIII (Invitrogen Corporation) for expression in insect cells. The resulting plasmid was termed pSX556.

In order to generate a recombinant baculovirus expressing HBP the MAXBAC kit from Invitrogen Corp. (San Diego, CA) was used and all manipulations were done according to the included Baculovirus Expression System Manual (version 1.5.5). Briefly, 1 µg og linearized AcMNPV DNA and 3 µg of pSX556 were co-transfected into SF9 insect cells (2 x 10⁶ cells in 60 mm dishes). The resulting culture supernatant was collected after 7 days. Fresh monolayers of SF9 cells in 100 mm plates were infected with virus supernatant at various dilutions and then overlaid with 1.5% agarose containing complete TNM-FH medium with 150 µg/ml X-gal. After 8 days 6 presumed recombinant plaques were identified by their blue collar and used to infect a 6 well plate containing SF9 cells. After 5 days the corresponding virus DNA was purified and subjected to a PCR reaction with forward and reverse primers flanking the site of recombination in the virus DNA. After evaluation of the PCR products on agarose gel the corresponding most pure recombinant virus was subjected to another round of plaque purification to ensure that the final recombinant virus stock was free of wildtype virus. The production of recombinant HBP was performed in insect cells (SF9 and SF21) adapted to growth in serum-free SF900-II medium (Gibco BRL/Life-Technologies). Typically, spinner cultures of 5 1 or a fermentor of 10 l, both types with a cell density of 1 x 10⁶/ml, were infected with a MOI of 1 and the medium was harvested 3 days post infection. Purification of HBP was carried out as described in WO 89/08666.

### Example 3

### HBP without proregion:

An oligonucleotide linker (see below) was made covering the first 99 bp of the sequence shown in Fig. 2 (from BamHI to Eag I) excluding the part covering the proregion (from 73 to 87, italics) and this was substituted for the original BamHI-Eag I in pSX556 giving rise to pSX559. This construction is expected to produce mature HBP when expressed in the Baculovirus system.

The linker consists of four oligonucleotides annealed pairwise to give the two following duplexes:

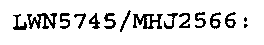

### Example 4

### Expression of HBP derivative with a bovine enterokinase cleavage site placed between the propeptide and mature HBP.

Insect cells infected with a baculovirus vector encoding HBP (signal-propeptide-mature HBP) are not able to cleave off the propeptide. Insect cells infected with virus encoding HBP without propeptide (signal sequence fused directly to mature HBP sequence) produce the mature form, but in very low amounts. In order to increase the yield of mature HBP a baculovirus vector encoding a HBP derivative with a bovine enterokinase cleavage site (Asp₄-Lys) inserted between the propeptide and mature HBP was generated, making it possible to obtain mature HBP by in vitro processing of the produced extended form of HBP:

A cDNA fragment encoding signal-Gly-Ser-Ser-Pro-Leu-Leu-Asp-Asp₃-Lys-mature HBP was generated by PCR with Pfu polymerase and the primers PBRa 241
(CCGGGGATCCGATGACCCGGCTGACAGTCCTGGCCCTGCTGGCTGGTCTGCTGGCGTCCTCG AGGGCCGGCTCCAGCCCCCTTTTGGACGACGACGACAAGATCGTTGGCGGC) and PBRa 246 (CCGGGGATCCAACTAGGCTGGCCCCGGTCCCGG). The PCR product was digested with BamHI and cloned into the pVL1393 transfer vector (Invitrogen, San Diego, CA). Generation of recombinant baculovirus and production of protein was done as described in Example 2 (however, in this case presumed recombinant plaques were identified by their occlusion-negative phenotype).

In order to produce the N-terminally extended form of HBP 4 x 10⁸ SF9 cells growing in SF900II serum-free medium (Gibco) were centrifuged down and resuspended in a sample from the virus stock giving a MOI (multiplicity of infection) of 1. The cells with virus were transferred to a 0.5 1 Bellco spinner flask (#1965-00500), and fresh SF900II medium was added to a final volume of 400 ml. Finally 1.5 g heparin-Sepharose (CL-6B, Pharmacia), which had been autoclaved in 25 ml sterile 0.9% NaCl, was added to the culture. The culture was incubated at 27°C for 3 days.

To isolate the heparin-Sepharose beads from the insect cell culture the 400 ml fermentation medium was aliquoted into 8 50 ml tubes and centrifuged in a Sorvall Instruments TECHNOSPIN R centrifuge at 300 rpm for 3 min. The supernatants with cells were sucked away and the pelleted heparin-Sepharose beads were separated from the remainder of contaminating cells by resuspension in 30 ml sterile NaCl added to each tube followed by centrifugation at 300 rpm. The entire procedure repeated twice. The beads were finally washed in 20 ml sterile 0.5 M NaCl added to each tube. The beads were then collected in one 50 ml tube in a small volumne of 0.5 M sterile NaCl (20-30 ml) and transferred to a sterile glass filter funnel. The beads were allowed to drain and the rHBP was finally eluted from the beads with 30 ml sterile 3 M NaCl. The HBP was purified from the 3 M NaCl eluate according to the method described in WO 89/08666.

Two hundred microgram purified HBP containing the prosequence and the enterokinase cleavage site between the prosequence and the mature protein was dissolved in 50 mM Na-acetate buffer containing 25 mM CaCl₂, pH 5.1. Four hundred units enterokinase from Biozyme Laboratories Limited GB Batch 18x2 Code EK3 was added in a volumne of 1.2 microliters and the mixture was incubated for 1 h at 37°C. HBP was finally purified from the incubation mixture by RP HPLC according to the method described in WO 89/08666. An aliquot of the purified HBP (1 nmol) was subjected to N-terminal sequencing which showed that the main sequence represents the correct mature HBP starting with an Ile indicating that the cleavage was successful.

### Example 5

### Expression of HBP derivatives with a Factor Xₐ cleavage site placed between the propeptide and mature HBP

An alternative strategy for the generation of mature HBP was the usage of factor Xₐ for in vitro cleavage of the produced material. The factor Xₐ recognition/cleavage site is Ile-Glu-Gly-Arg. By T.J. Gardella et al. (J. Biol.Chem., 26, 15854-15859, 1990) it has been reported that the placement of three small amino acids (Gly-Gly-Ser) just N-terminal to the factor Xₐ recognition site perhaps minimized possible steric hindrance effects and thereby the efficiency of factor Xₐ cleavage could be increased. In order to generate cDNA fragments encoding signal-propeptide-Ile-Glu-Gly-Arg-mature HBP and signal-propeptide-Gly-Gly-Ser-Ile-Glu-Gly-Arg-mature HBP, respectively, the two primers PBRa 249 and PBRa 250 were synthesized. Together with primer PBRa 246 (Example 4) these two primers were used in PCR reactions with Pfu polymerase. The two fragments were digested with BamHI and inserted into pVL1393. Generation of recombinant baculoviruses and production of proteins were done as described in Example 4.

### Example 6

Peripheral blood mononuclear cells (BMNC) were prepared from healthy adult blood donors (Blood Bank, Rigshospitalet) by centrifugation of citrate anticoagulated buffy coats on Lymphoprep® (Nycomed, Oslo, Norway). In some experiments, BMNCs were preincubated with PTX for 1 h before addition of lipopolysaccharide (LPS) (E.coli 055:B5; Difco Laboratories, Detroit, MI), final concentration 1 µg/ml, or PHA (Difco), final concentration 20 µg/ml, or purified protein derivative of tuberculin (PPD) (State Serum Institute, Copenhagen, Denmark), final concentration 25 µg/ml. The incubations were carried out in RPMI-1640 (BRL-Gibco, Roskilde, Denmark) containing 3% normal human serum (NHS), heat inactivated at 56°C for 30 min and supplemented with 0.8 mM glutamine, penicillin/streptomycin (BRL-Gibco, Denmark) 20 IE/ml each. After incubation, the cells were harvested and supernatants were immediately frozen at -20°C; the cell pellets were quickly frozen on liquid nitrogen and kept at -80°C for no more than 1 week before RNA was extracted.

IL-1α, IL-1β, IL-2, IL-6, IFNτ, TNFα and TNFβ were measured by a double-sandwish ELISA using monospecific polyclonal rabbit antibodies to purified recombinant cytokines (M.B.Hansen et al., Immunol. Lett. 30, 1991, p. 156). Immuno-Maxisorp plates (Nunc, Roskilde, Denmark) were coated with protein-A affinity-purified IgG. Non-attached sites were blocked with 5% human serum albumin in phosphate-buffered saline (PBS). Samples were diluted in PBS supplemented with 2% normal rabbit serum (Dako, Glostrup, Denmark), EDTA 10 mM, aprotinin 2,000 KIE/ml and 5 mM DL-dithiothreitol. The assays were calibrated with international standards of the respective cytokines (National Institute for Biological Standards and Controls, Potters Bar, Hertfordshire, UK). Biotinylated polyclonal rabbit antibodies were used as detecting antibodies along with streptavidin-peroxidase (Kirkegaard and Perry La., Gaithersburg, MD). Development was carried out with 1,2-phenylenediamine dihydrochloride and measured at 492 nm. The inter-assay coefficient of variation for the concentration range between 8 pg/ml and 1 ng/ml were less than 15%. The sensitivity limit of these ELISAs were 8-10 pg/ml.

The BMNCs were thawed and treated with human serum albumin in amounts of 10 µg/ml and 100 µg/ml, as well as HBP in amounts of 0.2 µg/ml, 2 µg/ml and 20 µg/ml in the presence of 10 and 100 ng/ml LPS, respectively.

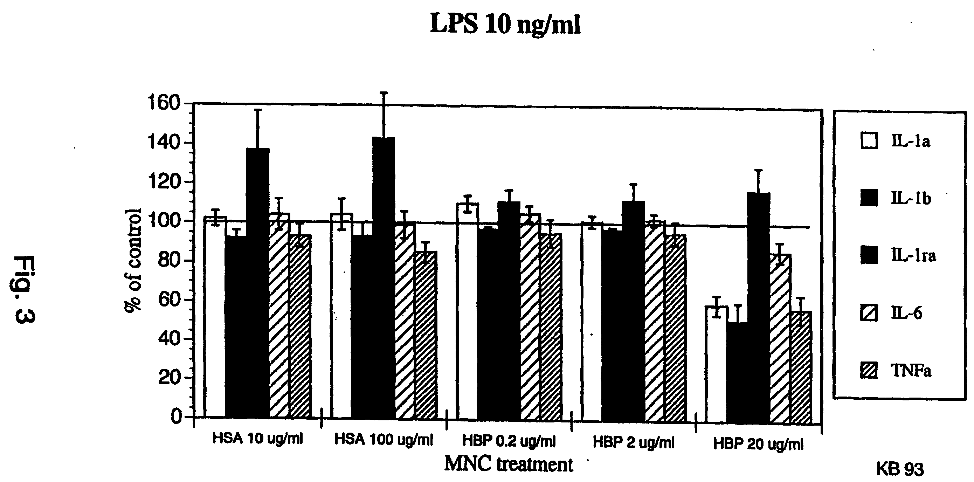

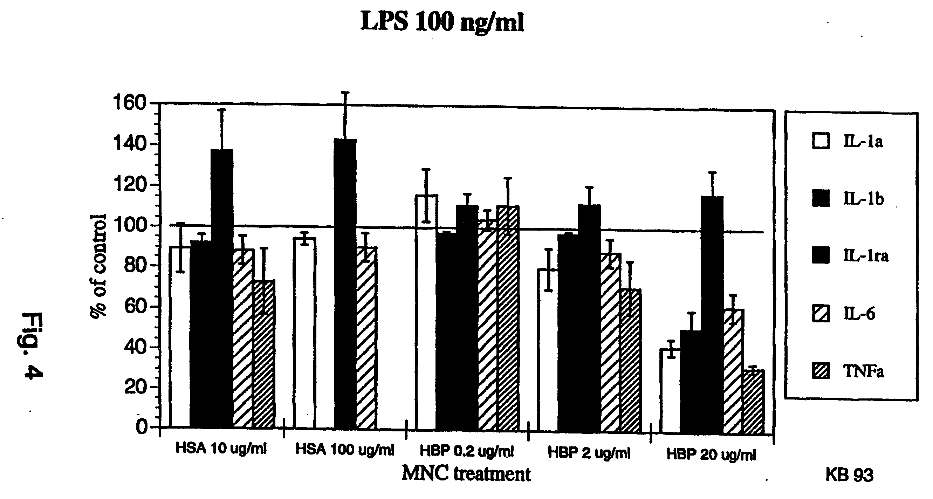

The results are shown in the appended Figs. 3 and 4. It appears from the figures that the release of cytokines from the BMNCs is significantly reduced in the presence of HBP.

## Claims

1. Use of heparin binding protein (HBP) which in glycosylated form has an apparent molecular weight of 28 kD (as determined by SDS-PAGE under reducing conditions) the HBP being producible in the azurophil granules of polymorphonuclear leukocytes or an analogue or a peptide fragment of HBP according to SEQ 1 or 2, the peptide being capable of binding the Lipid A portion of LPS, for the manufacture of a medicament for the prevention or treatment of sepsis, gram-negative sepsis, septic shock, or disseminated intravascular coagulation.

2. Use according to claim 1, wherein the medicament is in a form suited for local or systemic injection or infusion.

3. Use according to any one of the preceding claims, wherein the medicament is useful for local application at sites of inflammation or infection.

4. Use according to any one of the preceding claims, wherein the heparin-binding protein is human HBP.

5. Use according to any one of the preceding claims, wherein the HBP has the amino acid sequence shown in the Sequence Listing as SEQ ID NO:1, or an analogue or peptide fragment thereof capable of binding the lipid A portion of LPS.

6. Use according to any one of the preceding claims, wherein the peptide fragment capable of binding the lipid A portion of LPS is a peptide fragment at least comprising amino acid residues 20-53, in particular amino acid residues 26-42 of SEQ ID NO:1.

7. Use according to any one of the preceding claims, wherein the heparin-binding protein is porcine HBP.

8. Use according to any one of the preceding claims, wherein the HBP has the amino acid sequence shown in the Sequence Listing as SEQ ID NO:2 or an analogue or peptide fragment thereof capable of binding the lipid A portion of LPS.

9. Use according to any one of the preceding claims, wherein the peptide fragment capable of binding the lipid A portion of LPS is a peptide fragment at least comprising amino acid residues 20-53 of SEQ ID NO:2.

10. Use according to any one of the preceding claims, wherein the medicament contains HBP in an amount of from about 10 mg to about 1 g per unit dosage form.

## Patentansprüche

1. Verwendung von Heparin-bindendem Protein (HBP), welches in glykosilierter Form ein augenscheinliches Molekulargewicht von 28 kD hat (bestimmt mittels SDS-PAGE unter reduzierenden Bedingungen), welches in den azurophilen Granula von segmentkernigen Leukozyten produzierbar ist, oder einem Analogon oder einem Peptidfragment des HBP entsprechend der SEQ 1 oder 2, wobei das Peptid in der Lage ist an den Lipid A Teil von LPS zu binden, zur Herstellung eines Arzneimittels zur Prophylaxe oder Behandlung von Blutvergiftung, gram-negativer Sepsis, septischem Schock oder disseminierter intravasaler Gerinnung.

2. Verwendung gemäß Anspruch 1, wobei das Arzneimittel in einer Form vorliegt, welche für lokale oder systemische Injektion oder Infusion geeignet ist.

3. Verwendung gemäß einem der vorangegangenen Ansprüche, wobei das Arzneimittel für eine örtliche Applikation an einer Entzündungs- oder Infektionsstelle einsetzbar ist.

4. Verwendung gemäß einem der vorangegangenen Ansprüche, wobei das Heparin-bindende Protein das humane HBP ist.

5. Verwendung gemäß einem der vorangegangenen Ansprüche, wobei das HBP die Aminosäuresequenz, dargestellt in der Sequenzliste als SEQ ID Nr.: 1, oder ein Analogon oder Peptidfragment davon, welches in der Lage ist an den Lipid A Teil von LPS zu binden, hat.

6. Verwendung gemäß einem der vorangegangenen Ansprüche, wobei das Peptidfragment, welches in der Lage ist an den Lipid A Teil von LPS zu binden, wenigstens die Aminosäurenreste 20-53, insbesondere die Aminosäurereste 26-42 der SEQ ID Nr.: 1, umfaßt.

7. Verwendung gemäß einem der vorangegangenen Ansprüche, wobei das Heparin-bindende Protein das aus Schweinen stammende HBP ist.

8. Verwendung gemäß einem der vorangegangenen Ansprüche, wobei das HBP die Aminosäuresequenz, dargestellt in der Sequenzliste als SEQ ID Nr.: 2, oder ein Analogon oder Peptidfragment davon, welches in der Lage ist an den Lipid A Teil von LPS zu binden, hat.

9. Verwendung gemäß einem der vorangegangenen Ansprüche, wobei das Peptidfragment, welches in der Lage ist an den Lipid A Teil von LPS zu binden, wenigstens die Aminosäurenreste 20-53 der SEQ ID Nr.: 2 umfaßt.

10. Verwendung gemäß einem der vorangegangenen Ansprüche, wobei das Arzneimittel eine Menge von ungefähr 10 mg bis zu ungefähr 1 g HBP pro Einzeldosis enthält.

## Revendications

1. Utilisation de la protéine liant l'héparine (PLH) qui, sous forme glycosylée a un poids moléculaire apparent de 28 kD (déterminé par EGPA-SDS dans des conditions réductrices) la PLH étant obtenue dans des granules azurophiles de leucocytes polynucléaires ou un analogue ou un fragment peptidique de la PLH selon la séquence 1 ou 2, le peptide étant capable de lier la portion de lipide A de la PLH, pour la production d'un médicament destiné à la prévention ou au traitement de la septicémie, de la septicémie à gram-négatifs, du choc septique ou de la coagulation intravasculaire disséminée.

2. Utilisation selon la revendication 1, dans laquelle le médicament est sous une forme adaptée à l'injection locale ou systémique ou à la perfusion.

3. Utilisation selon l'une quelconque des revendications précédentes, dans laquelle le médicament est utile pour l'application locale sur les sites d'inflammation ou d'infection.

4. Utilisation selon l'une quelconque des revendications précédentes, dans laquelle la protéine liant l'héparine est la PLH humaine,

5. Utilisation selon l'une quelconque des revendications précédentes, dans laquelle la PLH possède la séquence d'acides aminés indiquée dans la liste de séquences sous le NO ID SEQ.1, ou un analogue ou un de ses fragments peptidiques capables de lier la portion de lipide A de la PLH.

6. Utilisation selon l'une quelconque des revendications précédentes, dans laquelle le fragment peptidique capable de lier la portion de lipide A de la PLH est un fragment peptidique comprenant au moins les résidus d'acides aminés 20-53, en particulier les résidus d'acides aminés 26-42 de NO ID SEQ:1.

7. Utilisation selon l'un quelconque des revendications précédentes, dans laquelle la protéine liant l'héparine est la PLH porcine.

8. Utilisation selon l'une quelconque des revendications précédentes, dans laquelle la PLH possède la séquence d'acides aminés indiquée dans la liste de séquences sous le NO ID SEQ: 2 ou un analogue ou un de ses fragments peptidiques capables de lier la portion de lipide A de la PLH.

9. Utilisation selon l'une quelconque des revendications précédentes, dans laquelle le fragment peptidique capable de lier la portion de lipide A de la PLH est un fragment peptidique comprenant au moins les résidus d'acides aminés 20-53 du NO ID SEQ:2

10. Utilisation selon l'une quelconque des revendications précédentes, dans laquelle le médicament contient la PLH à une teneur comprisc ente 10 mg et environ 1 g par dose unitaire.
